# EUROPÄISCHE PATENTSCHRIFT

(11) **EP 2 997 010 B1**
(45) Veröffentlichungstag und Bekanntmachung des Hinweises auf die Patenterteilung: **15.11.2017**
(21) Anmeldenummer: 14722684.9
(22) Anmeldetag: 09.05.2014
(51) Int. Cl.: C07C 273/18, C07C 275/40, C08K 5/29

(54) **NEUE CARBODIIMIDE MIT ENDSTÄNDIGEN HARNSTOFF- UND/ODER URETHANGRUPPEN, VERFAHREN ZU DEREN HERSTELLUNG UND DEREN VERWENDUNG**
NOVEL CARBODIIMIDES WITH UREA AND/OR URETHANE TERMINAL GROUPS, PROCESS FOR THEIR PREPARATION AND THEIR USE
NOUVEAUX CARBODIIMIDES COMPRENANT DES GROUPES URÉE ET/OU URÉTHANE À TERMINAISON, LEUR PROCÉDÉ DE FABRICATION ET LEUR UTILISATION

(30) Priorität: 13.05.2013 EP 13167511
(43) Veröffentlichungstag der Anmeldung: 23.03.2016
(73) Patentinhaber: LANXESS Deutschland GmbH, 50569 Köln (DE)
(72) Erfinder: LAUFER, Wilhelm, 67158 Ellerstadt (DE); BECHEM, Benjamin, 68165 Mannheim (DE); ECKERT, Armin, 68794 Oberhausen-Rheinhausen (DE)
(86) Internationale Anmeldenummer: PCT/EP2014/059578
(87) Internationale Veröffentlichungsnummer: WO 2014/184116

(56) Entgegenhaltungen:
- EP-A1- 0 628 541
- WO-A1-2005/111137
- DE-A1- 2 248 751
- DE-A1-102004 041 605
- US-A- 2 941 983
- DANIEL W. BROWN ET AL: "Kinetics of the reaction between polyester acid and carbodiimide in dry polyester diols and in a polyester polyurethane", MACROMOLECULES, Bd. 14, Nr. 3, 1. Mai 1981 (1981-05-01), Seiten 659-663, XP055080186, ISSN: 0024-9297, DOI: 10.1021/ma50004a040
- CAMPBELL T W ET AL: "CARBODIIMIDES. IV. HIGH POLYMERS CONTAINING THE CARBODIIMIDE REPEATUNIT", THE JOURNAL OF ORGANIC CHEMISTRY, AMERICAN CHEMICAL SOCIETY [NOT]ETC., Bd. 28, 1. August 1963 (1963-08-01), Seiten 2069-2075, XP000673148, ISSN: 0022-3263, DOI: 10.1021/JO01043A029

## Beschreibung

Die Erfindung betrifft neue Carbodiimiden mit endständigen Harnstoff- und/oder Urethangruppen, Verfahren zu deren Herstellung und deren Verwendung als Stabilisator in esterbasierten Polyolen, in Polyethylenterephthalat (PET), in Polybutylenterephthalat (PBT), in Polytrimethylenterephthalat (PTT), in Copolyestern, in thermoplastischen Polyester-Elastomeren (TPE E), in Ethylenvinylacetat (EVA), in Polymilchsäure (PLA) und/oder in PLA-Derivaten, in Polybutylenadipat-Terephthalaten (PBAT), in Polybutylensuccinaten (PBS), in Polyhydroxyalkanoaten (PHA), in Blends, in Triglyceriden, in thermoplastischen Polyurethanen, in Polyurethan-Elastomeren, in PU-Klebstoffen, in PU-Gießharzen, für PU-Beschichtungen oder in PU-Schäumen.

Carbodiimide haben sich in vielen Anwendungen bewährt, z.B. als Hydrolyseschutzmittel für Thermoplasten, Polyole, Polyurethane, Triglyceride und Schmierstofföle etc.

Bevorzugt werden hierfür sterisch gehinderte aromatische Monocarbodiimide eingesetzt. Bekannt ist in diesem Zusammenhang vor allem 2,6-Diisopropylphenyl-Carbodiimid. Diese Monocarbodiimide haben jedoch den Nachteil, selbst bei niedrigen Temperaturen flüchtig zu sein. Sie sind thermisch nicht stabil und können giftige, flüchtige Substanzen abspalten. Weitere Carbodiimide, wie in EP 0 628 541 A1 beschrieben, basieren auf speziellen Rohstoffen, die sehr teuer in der Anschaffung sind. Zudem haben diese bei Raumtemperatur hohe Viskositäten, die den Umgang mit diesen Carbodiimiden erschweren. Weiterhin ist in bestimmten PU-, PET-, PLA- oder Schmierstoff-Anwendungen deren Reaktivität und/oder deren stabilisierende Wirkung bei den standardmäßig eingesetzten Konzentrationen nicht ausreichend. Polymere Carbodiimide, die auf preiswerten Rohstoffen basieren, wie in DE-A 2248751 und US-A 2941983 beschrieben, sind nicht ausreichend sterisch gehindert und zeigen in den meisten esterbasierten Polymeren keine gute Hydrolyseschutzwirkung. Stark sterisch gehinderte Carbodiimide, wie z.B. solche auf Basis Triisopropylphenylisocyanat, sind sehr wirksam, haben jedoch sehr hohe Schmelzpunkte, sind nicht löslich und können nicht oder nur mit einem beträchtlichen apparativen und zeitlichen Aufwand in die Ausgangsstoffe der Polyurethane eingebracht werden.

Es besteht daher ein Bedarf an neuen Carbodiimiden, welche die Nachteile des Standes der Technik nicht aufweisen, über eine hohe thermische Stabilität verfügen und zum Hydrolyseschutz esterbasierter Polymere eingesetzt werden können.

Überraschenderweise konnte diese Aufgabe durch den Einsatz von bestimmten aromatischen Carbodiimiden gelöst werden.

Gegenstand der vorliegenden Erfindung sind daher Carbodiimide mit endständigen Harnstoff- und oder Urethangruppen der Formel (I) 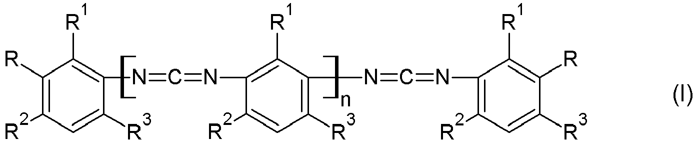 in der
- R gleich oder verschieden sein kann und ausgewählt ist aus der Gruppe der -NHCONHR^{I}-, -NHCONR^{I}R^{II}- und -NHCOOR^{III}-Reste, wobei R^{I} und R^{II} gleich oder verschieden sind und einen C₁ - C₂₂ - Alkyl-, C₆ - C₁₂ -Cycloalkyl-, C₆ - C₁₈ -Aryl- oder C₆ - C₁₈ -Aralkylrest bedeuten und R^{III} gleich C₁ - C₂₂ - Alkyl, bevorzugt C₁ - C₆ - Alkyl, besonders bevorzugt Methyl, Ethyl oder i-Propyl, C₆ - C₁₂ - Cycloalkyl, bevorzugt C₆ -Cycloalkyl, C₆ - C₁₈ -Aryl oder C₆ - C₁₈ -Aralkyl, sowie einen ungesättigten Alkylrest (z.B. einen Oleylrest) mit 2 - 22, bevorzugt 12 - 20, besonders bevorzugt 16 - 18 Kohlenstoffatomen, oder ein Alkoxypolyoxyalkylenrest bedeutet,
- R¹, R² und R³ jeweils unabhängig für Methyl- oder Ethyl steht, wobei jeder Benzolring nur eine Methyl-Gruppe aufweist und
- n = 0 bis 20, bevorzugt n = 1 bis 10 ist.

Der Carbodiimid-Gehalt (NCN-Gehalt, gemessen durch Titration mit Oxalsäure) der erfindungsgemäßen Carbodiimide liegt vorzugsweise bei 2 - 14 Gew.%.

Bevorzugt sind Carbodiimide der Formel (I) mit R = -NHCOOR^{III}, wobei R^{III} ein Alkoxypolyoxyalkylenrest ist, R¹, R² und R³ jeweils unabhängig für Methyl- oder Ethyl steht, wobei jeder Benzolring nur eine Methyl-Gruppe aufweist und n = 0 bis 20, bevorzugt n = 1 bis 10, besonders bevorzugt n = 1 bis 4, ganz besonders bevorzugt n = 2 bis 3 ist. Der Carbodiimid-Gehalt dieser bevorzugten Carbodiimide liegt vorzugsweise bei 2 - 10 Gew.%, besonders bevorzugt bei 4 - 8 Gew.%, ganz besonders bevorzugt bei 5 - 7 Gew.%.

Bevorzugte Alkoxypolyoxyalkylenreste sind Polyethylenglykolmonomethylether mit Molmassen von 200 - 600 g/mol, besonders bevorzugt von 350 - 550 g/mol.

Ebenfalls bevorzugt sind Carbodiimide der Formel (I) mit R = -NHCOOR^{III}, wobei R^{III} gleich C₁ - C₂₂ - Alkyl, bevorzugt C₁ - C₆ - Alkyl, besonders bevorzugt Methyl, Ethyl oder i-Propyl, C₆ - C₁₂ -Cycloalkyl, bevorzugt C₆ -Cycloalkyl ist und R¹, R² und R³ jeweils unabhängig für Methyl- oder Ethyl steht, wobei jeder Benzolring nur eine Methyl-Gruppe aufweist und n = 0 bis 20, bevorzugt n = 1 bis 10, besonders bevorzugt n = 3 bis 8 ist. Der Carbodiimid-Gehalt dieser bevorzugten Carbodiimide liegt vorzugsweise bei 4 - 13 Gew.%, besonders bevorzugt bei 10 - 13 Gew.%.

Diese bevorzugten Carbodiimide der Formel (I) mit R = -NHCOOR^{III}, wobei R^{III} gleich C₁ - C₂₂ - Alkyl, bevorzugt C₁ - C₆ - Alkyl, besonders bevorzugt Methyl, Ethyl oder i-Propyl, C₆ - C₁₂ -Cycloalkyl, bevorzugt C₆ -Cycloalkyl ist und R¹, R² und R³ jeweils unabhängig für Methyl- oder Ethyl steht, wobei jeder Benzolring nur eine Methyl-Gruppe aufweist und n = 0 bis 20, bevorzugt n = 1 bis 10, besonders bevorzugt n = 3 bis 8, sind fest und weisen Erweichungspunkte > 40°C auf und sind daher hervorragend für die Stabilisierung der esterbasierten Polymere geeignet, die vorzugsweise ausgewählt sind aus der Gruppe der Polyethylenterephthalat (PET), Polybutylenterephthalat (PBT), Polytrimethylenterephthalat (PTT), thermoplastische Polyurethane (TPU), Copolyester, wie das modifizierte Polyester aus Cyclohexandiol und Terephthalsäure (PCTA), thermoplastische Polyester Elastomere (TPE E), Ethylenvinylacetat (EVA), Polymilchsäure (PLA) und/oder PLA-Derivate, Polyhydroxyalkanoate (PHA), Polybutylenadipat-Terephthalat (PBAT), Polybutylensuccinat (PBS), oder in Blends, wie z.B. PA/PET- oder PHA/PLA-Blends geeignet, was ebenfalls Gegenstand der vorliegenden Erfindung ist.

Für das erfindungsgemäße Verfahren zur Stabilisierung der esterbasierten Polymere werden die Carbodiimide der Formel (I) mit R = -NHCOOR^{III}, wobei R^{III} gleich C₁ - C₂₂ - Alkyl, bevorzugt C₁- C₆ - Alkyl, besonders bevorzugt Methyl, Ethyl oder i-Propyl, C₆ - C₁₂-Cycloalkyl, bevorzugt C₆ -Cycloalkyl ist und R¹, R² und R³ jeweils unabhängig für Methyl- oder Ethyl steht, wobei jeder Benzolring nur eine Methyl-Gruppe aufweist und n = 0 bis 20, bevorzugt n = 1 bis 10, besonders bevorzugt n = 3 bis 8, zu den esterbasierten Polymeren ausgewählt aus der Gruppe, umfassend Polyethylenterephthalat (PET), Polybutylenterephthalat (PBT), Polytrimethylenterephthalat (PTT), thermoplastische Polyurethane (TPU), Copolyester, wie das modifizierte Polyester aus Cyclohexandiol und Terephthalsäure (PCTA), thermoplastische Polyester Elastomere (TPE E), Ethylenvinylacetat (EVA), Polybutylenadipat-Terephthalat (PBAT), Polybutylensuccinat (PBS), Polymilchsäure (PLA) und/oder PLA-Derivate, Polyhydroxyalkanoate (PHA), oder in Blends, wie z.B. PA/PET- oder PHA/PLA-Blends mittels Feststoffdosieraggregaten zugegeben.

Feststoffdosieraggregate im Sinne der Erfindung sind vorzugsweise: Ein-, Zwei- und Mehrwellenextruder, kontinuierlich arbeitenden Co-Kneter (Typ Buss) und diskontinuierlich arbeitenden Kneter, z.B Typ Banbury und andere in der Polymerindustrie üblichen Aggregate.

Die Konzentration der erfindungsgemäßen Carbodiimiden der Formel (I) in den esterbasierten Polymeren ist vorzugsweise 0,1 - 5 Gew.%, bevorzugt 0,5 - 3 Gew.%, besonders bevorzugt 1 - 2 Gew.%.

In einer weiteren Ausführung der Erfindung sind solche Carbodiimide der Formel (I) bevorzugt, in denen R gleich -NHCOOR^{III} ist, wobei R^{III} ein gesättigter und/oder ungesättigter Alkylrest mit 2 - 22, bevorzugt 12 - 20, besonders bevorzugt 16 - 18 Kohlenstoffatomen ist, und R¹, R² und R³ jeweils unabhängig für Methyl- oder Ethyl steht, wobei jeder Benzolring nur eine Methyl-Gruppe aufweist und n = 0 bis 20, bevorzugt n = 1 bis 10, besonders bevorzugt n = 1 bis 4, ganz besonders bevorzugt n = 2 bis 3 ist. Der Carbodiimid-Gehalt dieser bevorzugten Carbodiimide liegt vorzugsweise bei 2 - 10 Gew.%, besonders bevorzugt bei 4 - 8 Gew.%, ganz besonders bevorzugt bei 5 - 7 Gew.%.

Die erfindungsgemäßen Carbodiimide weisen darüber hinaus vorzugsweise mittlere Molmassen (Mw) von 1000 - 5000 g/mol, bevorzugt 1500 - 4000 g/mol, besonders bevorzugt 2000 - 3000 g/mol auf.

Weiterhin sind Carbodiimide bevorzugt, die eine Polydispersität D = Mw/Mn von 1,2 - 2, besonders bevorzugt von 1,4 - 1,8 aufweisen.

Der Rahmen der Erfindung erfasst alle oben stehenden und im Folgenden aufgeführten allgemeinen oder in Vorzugsbereichen genannten Restedefinitionen, Indizes, Parameter und Erläuterungen untereinander, also auch zwischen den jeweiligen Bereichen und Vorzugsbereichen in beliebiger Kombination.

Gegenstand der vorliegenden Erfindung ist zudem die Herstellung der erfindungsgemäßen Carbodiimide durch die Carbodiimidisierung von aromatischen Diisocyanaten der Formel (II) 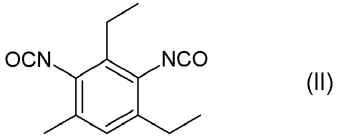 und/oder der Formel (III) 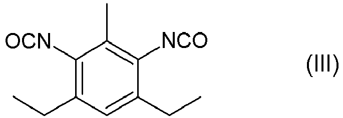 unter Abspaltung von Kohlendioxid bei Temperaturen von 80 °C bis 200 °C in Gegenwart von Katalysatoren und gegebenenfalls Lösungsmittel und der anschließenden Endfunktionalisierung der freien NCO-Gruppen mit primären oder sekundären Aminen oder Alkoholen und/oder Alkoxypolyoxyalkylenalkoholen.

Bevorzugt werden Mischungen der Diisocyanate der Formeln (II) und (III) eingesetzt, vorzugsweise im Verhältnis 60 : 40 bis 95 : 5, besonders bevorzugt 70 : 30 bis 90 : 10.

Die für die Herstellung der Diisocyanate notwendigen aromatischen Diamine können - wie dem Fachmann bekannt- über eine Friedel-Crafts-Alkylierung der entsprechenden Toluoldiamine mit dem entsprechenden Alken, oder Halogenalkan hergestellt werden. Bei den aromatischen Diaminen handelt es sich um handelsübliche Verbindungen, die z.B. bei der Firma Lonza AG unter dem Handelsnamen Lonzacure® erhältlich sind.

Anschließend werden diese Diamine mit Phosgen zum entsprechenden Diisocyanat umgesetzt.

Zur Herstellung der erfindungsgemäßen Carbodiimide können die Diisocyanate der Formel (II) und/oder (III) bei erhöhter Temperaturen, vorzugsweise Temperaturen von 80 - 200 °C, besonders bevorzugt von 100 bis 180°C, ganz besonders bevorzugt von 120 - 140°C, zweckmäßigerweise in Gegenwart von Katalysatoren unter Kohlendioxidabspaltung kondensiert werden. Hierfür geeignete Verfahren werden beispielsweise beschrieben in DE-A 1130594 und DE-A 11564021.

Als Katalysatoren für die Herstellung der Verbindungen der Formel (I) sind in einer Ausführungsform der Erfindung Phosphorverbindungen bevorzugt. Als Phosphorverbindungen werden vorzugsweise Phospholenoxide, Phospholidine oder Phospholinoxide sowie die entsprechenden Phospholensulfide verwendet. Ferner können als Katalysatoren tertiäre Amine, basisch reagierende Metallverbindungen, Alkali-, Erdalkalioxide oder -Hydroxide, -Alkoholate oder -Phenolate, Carbonsäuremetallsalze und nicht basische Organometallverbindungen verwendet werden.

Die Carbodiimidisierung kann sowohl in Substanz als auch in einem Lösemittel durchgeführt werden. Als Lösemittel werden vorzugsweise Alkylbenzole, Paraffinöle, Polyethylenglykoldimethylether, Ketone oder Lactone eingesetzt.

Wenn die Reaktionsmischung den gewünschten Gehalt an NCO-Gruppen, entsprechend einem mittleren Kondensationsgrad von n = 0 bis 20, bevorzugt n = 1 bis 10 besitzt wird die Polycarbodiimidisierung üblicherweise gestoppt.

In einer Ausführungsform der vorliegenden Erfindung wird hierzu die Temperatur der Reaktionsmischung auf 50 - 120 °C, bevorzugt 60 - 100 °C, besonders bevorzugt auf 80 - 90 °C reduziert und die Katalysatoren werden unter verminderten Druck abdestilliert. In einer bevorzugten Herstellungsvariante der erfindungsgemäßen Carbodiimide wird anschließend das überschüssige Diisocyanat bei Temperaturen von 150 - 200°C, bevorzugt 160 - 180°C abdestilliert. Anschließend werden die freien endständigen Isocyanatgruppen der Carbodiimide mit aliphatischen und/oder aromatischen Aminen, Alkoholen und/oder Alkoxypolyoxyalkylenalkoholen vorzugsweise in einem geringem Überschuss an -NH-, -NH₂- und/oder -OH-Gruppen, gegebenenfalls in Anwesenheit eines dem Fachmann bekannten PU-Katalysators, vorzugsweise tert. Aminen oder Organozinn-Verbindungen, besonders bevorzugt DBTL (Dibutylzinndilaurat oder DOTL (Dioctylzinndilaurat), abreagiert. Das Stoffmengen-Verhältnis von Aminen, Alkoholen und/oder Alkoxypolyoxyalkylenalkoholen zu Carbodiimiden liegt vorzugsweise bei 1,005 - 1,05 : 1, besonders bevorzugt bei 1,01 - 1,03 : 1, bezogen auf die vorhandenen N=C=O-Gruppen.

Als Alkohole sind Ethanol und Cyclohexanol bevorzugt.

In einer weiteren Ausführungsform der vorliegenden Erfindung wird zur Unterbrechung der Carbodiimidisierung die Temperatur der Reaktionsmischung auf 50 - 120 °C, bevorzugt 60 - 100 °C, besonders bevorzugt auf 80 - 90 °C reduziert und gegebenenfalls nach Zugabe eines Lösemittels, bevorzugt ausgewählt aus der Gruppe der Alkylbenzole, besonders bevorzugt Toluol, werden die freien endständigen Isocyanatgruppen der Carbodiimide mit aliphatischen und/oder aromatischen Aminen, Alkoholen und/oder Alkoxypolyoxyalkylenalkoholen vorzugsweise in einem geringem Überschuss an -NH-, -NH₂- und/oder -OH-Gruppen, gegebenenfalls in Anwesenheit eines dem Fachmann bekannten PU-Katalysators, vorzugsweise tert. Aminen oder Organozinn-Verbindungen, besonders bevorzugt DBTL (Dibutylzinn dilaurat oder DOTL (Dioctylzinndilaurat), abreagiert. Das Stoffmengen-Verhältnis von Aminen, Alkoholen und/oder Alkoxypolyoxyalkylenalkoholen zu Carbodiimiden liegt vorzugsweise bei 1,005 - 1,05 : 1, besonders bevorzugt bei 1,01 - 1,03 : 1, bezogen auf die vorhandenen N=C=O-Gruppen.

Nach vollständiger Reaktion wird der Katalysator und gegebenenfalls das Lösemittel vorzugsweise bei Temperaturen von 80 - 200°C unter verminderten Druck abdestilliert.

Als Alkohole sind Ethanol und Cyclohexanol bevorzugt.

Gegenstand der vorliegenden Erfindung ist zudem ein weiteres Verfahren zur Herstellung der erfindungsgemäßen Carbodiimide durch eine partielle, vorzugsweise < 50% ige Endfunktionalisierung der freien NCO-Gruppen mit primären oder sekundären Aminen oder Alkoholen und/oder Alkoxypolyoxyalkylenalkoholen in den aromatischen Diisocyanaten der Formel (II) 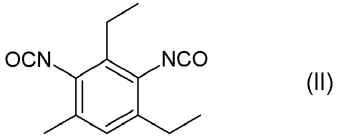 und/oder der Formel (III) 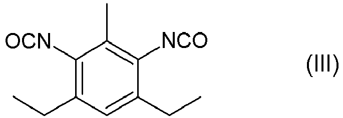 und anschließende Carbodiimidisierung unter Abspaltung von Kohlendioxid bei Temperaturen von 80 °C bis 200 °C in Gegenwart von Katalysatoren und gegebenenfalls Lösungsmittel.

Vorzugsweise werden die erfindungsgemäßen Carbodiimide nach ihrer Herstellung gereinigt. Die Reinigung der Rohprodukte kann destillativ und/oder mittels Extraktion mit Lösemitteln erfolgen. Als geeignete Lösemittel für die Aufreinigung können vorzugsweise Polyethylenglykoldimethylether, Alkylbenzole, Paraffinöle, Alkohle, Ketone oder Ester eingesetzt werden. Dabei handelt es sich um handelsübliche Lösemittel.

Gegenstand der vorliegenden Erfindung ist weiterhin eine Zusammensetzung enthaltend
- mindestens ein esterbasiertes Polymer,
   und
- mindestens ein erfindungsgemäßes Carbodiimid der Formel (I).

Bei den esterbasierten Polymeren handelt es sich vorzugsweise um Polyesterpolyole, esterbasierte thermoplastische Polyurethane, esterbasierte Polyurethan-Elastomere oder -Schäume, Polyethylenterephthalat (PET), Polybutylenterephthalat (PBT), Polytrimethylenterephthalat (PTT), Copolyester, wie das modifizierte Polyester aus Cyclohexandiol und Terephthalsäure (PCTA), thermoplastische Polyester Elastomere (TPE E), Ethylenvinylacetat (EVA), Polymilchsäure (PLA) und/oder PLA-Derivate, Polyhydroxyalkanoate (PHA), Polybutylenadipat-Terephthalat (PBAT), Polybutylensuccinat (PBS) oder in Blends, wie vorzugsweise PA/PET- oder PHA/PLA-Blends. Hierbei handelt es sich um kommerziell erhältliche Polymere.

In einer besonders bevorzugten Ausführungsform der Erfindung handelt es sich bei den Estergruppen-enthaltenden Polymeren um Polymilchsäure (PLA).

Dabei beträgt die Konzentration des erfindungsgemäßen Carbodiimides der Formel (I) in der erfindungsgemäßen Zusammensetzung vorzugsweise 0,1 - 5 Gew.%, bevorzugt 0,5 - 3 Gew.%, besonders bevorzugt 1 - 2 Gew.%.

Bei den Polyesterpolyolen als esterbasierte Polymere handelt es sich vorzugsweise um langkettige Verbindungen, die vorzugsweise ein Molekulargewicht (in g/mol) von bis zu 2000, bevorzugt zwischen 500 - 2000 und besonders bevorzugt zwischen 500 - 1000, aufweisen.

Der Begriff Polyesterpolyole im Sinne der Erfindung umfasst dabei sowohl langkettige Diole als auch Triole, wie auch Verbindungen mit mehr als drei Hydroxylgruppen je Molekül.

Vorteilhaft ist es, wenn das Polyesterpolyol eine OH-Zahl von bis zu 200, vorzugsweise zwischen 20 und 150 und besonders bevorzugt zwischen 50 und 115, aufweist. Insbesondere eignen sich Polyesterpolyole, die Reaktionsprodukte von verschiedenen Polyolen mit aromatischen oder aliphatischen Dicarbonsäuren und/oder Polymere von Lactonen sind.

Bei den im Sinne der Erfindung eingesetzten Polyesterpolyolen handelt es sich um handelsübliche Verbindungen, die bei der Firma Bayer MaterialScience AG unter dem Handelsnamen Baycoll® oder Desmophen® erhältlich sind.

Ein weiterer Gegenstand der vorliegenden Erfindung ist zudem ein bevorzugtes Verfahren zur Herstellung der erfindungsgemäßen Carbodiimide der Formel (I) mit R = -NHCOOR^{III}, wobei R^{III} gleich C₁ - C₂₂ - Alkyl, bevorzugt C₁ - C₆ - Alkyl, besonders bevorzugt Methyl, Ethyl oder i-Propyl, C₆ - C₁₂ -Cycloalkyl, besonders bevorzugt C₆ - Cycloalkyl ist und R¹, R² und R³ jeweils unabhängig für Methyl- oder Ethyl steht, wobei jeder Benzolring nur eine Methyl-Gruppe aufweist und n = 0 bis 20, bevorzugt n = 1 bis 10, besonders bevorzugt n = 3 bis 8 ist, bei dem die Schmelze nach der Carbodiimidisierung und gegebenenfalls Aufreinigung bevorzugt auf Pastillierbändern pastilliert wird. Dabei sind gängige Pastilliersysteme ebenso wie gängige Granuliersysteme einsetzbar. Diese sind z.B. erhältlich bei der Firma Sandvik Holding GmbH oder der Firma GMF Gouda.

Ganz besonders geeignet sind die erfindungsgemäßen Carbodiimide der Formel (I) mit R = -NHCOOR^{III}, wobei R^{III} Cyclohexyl ist.

Gegenstand der vorliegenden Erfindung ist zudem die Verwendung der erfindungsgemäßen Carbodiimide in esterbasierten Polyolen, Polyethylenterephthalat (PET), Polybutylenterephthalat (PBT), Polytrimethylenterephthalat (PTT), Copolyestern, wie das modifizierte Polyester aus Cyclohexandiol und Terephthalsäure (PCTA), thermoplastischen Polyester Elastomeren (TPE E), Ethylenvinylacetat (EVA), Polymilchsäure (PLA) und/oder PLA-Derivaten, Polyhydroxyalkanoaten (PHA), Polybutylenadipat-Terephthalat (PBAT), Polybutylensuccinat (PBS), in Blends, wie z.B. PA/PET- oder PHA/PLA-Blends, in Triglyceriden, vorzugsweise Trimethylolpropantrioleat (TMP-Oleat), in Ölformulierungen für die Schmierstoffindustrie, in thermoplastischen Polyurethanen (TPU), in Polyurethan-Elastomeren, in PU-Klebstoffen, in PU-Gießharzen, in PU-Schäumen oder in PU-Beschichtungen für Holz, Leder, Kunstleder und Textilien als Schutz gegen hydrolytischen Abbau. Dabei ist die Verwendung in Polymilchsäure (PLA) besonders bevorzugt.

Die nachfolgenden Beispiele dienen der Erläuterung der Erfindung.

### Ausführungsbeispiele

### Getestet wurden:

1) CDI (A): ein flüssiges Carbodiimid mit einem NCN-Gehalt von ca. 7,5 Gew.%, auf Basis von 1,3-Bis-(1-methyl-1-isocyanatoethyl)-benzol, endfunktionalisiert mit Polyethylenglykolmonomethylether PEG 550 MME, hergestellt analog zu Beispiel 2 in EP-A 0 628 541, Vergleich.
2) CDI (B): ein flüssiges Carbodiimid der Formel (I) mit einem NCN-Gehalt von ca. 6 Gew.%, n = ca. 2, erhalten durch Umsetzung einer Mischung auf Basis ca. 80 Gew.% von 2,4-Diethyltoluoyldiisocyanat, d.h. des Diisocyanats der Formel (II), und 20 Gew.% von 2,6-Diethyltoluoyldiisocyanat, d.h. des Diisocyanats der Formel (III), endfunktionalisiert mit Polyethylenglykol-monomethylether (PEG 550 MME der Firma BASF SE), erfindungsgemäß.
3) CDI (C): ein hochviskoses Carbodiimid mit einem NCN-Gehalt von ca. 14 Gew.%, auf Basis von 80 Gew.% von 2,4-Diethyltoluoyldiisocyanat, d.h. des Diisocyanats der Formel (II), und 20 Gew.% von 2,6-Diethyltoluoyldiisocyanat, d.h. des Diisocyanats der Formel (III) mit n > 40, nicht endfunktionalisiert (Vergleich).
4) CDI (D): ein festes polymeres Carbodiimid mit einem NCN-Gehalt von ca. 13,5 Gew.% auf Basis von Triisopropylphenyldiisocyanat, endfunktionalisiert (Vergleich), erhältlich bei der Rhein Chemie Rheinau GmbH unter dem Namen Stabaxol ® P.
5) CDI (E): ein monomeres festes Carbodiimid mit einem NCN-Gehalt von ca. 10,8 Gew.% auf Basis von 2,6-Diisopropylphenylisocyanat (Vergleich), erhältlich bei der Rhein Chemie Rheinau GmbH unter dem Namen Stabaxol ® I.
6) CDI (F): ein festes Carbodiimid mit einem NCN-Gehalt von ca. 12,5 Gew.%, auf Basis ca. 80 Gew.% von 2,4-Diethyltoluoyldiisocyanat, d.h. des Diisocyanats der Formel (II), und 20 Gew.% von 2,6-Diethyltoluoyldiisocyanat, d.h. des Diisocyanats der Formel (III) endfunktionalisiert mit Ethanol mit n= ca. 6, erfindungsgemäß.
7) CDI (G): ein festes Carbodiimid mit einem NCN-Gehalt von ca. 10,2 Gew.%, auf Basis ca. 80 Gew.% von 2,4-Diethyltoluoyldiisocyanat, d.h. des Diisocyanats der Formel (II), und 20 Gew.% von 2,6-Diethyltoluoyldiisocyanat, d.h. des Diisocyanats der Formel (III) endfunktionalisiert mit Cyclohexanol mit n= ca. 5, erfindungsgemäß.

### Esterbasierte Polymere:

8) Polyesterpolyol auf Basis von Adipinsäure (Desmophen® 2001 KS der Bayer MaterialScience AG).
9) Polyethylenterephthalat (PET) erhältlich bei der Firma Novapet.
10) Polymilchsäure (PLA, Spritzgussqualität) erhältlich bei der Firma NatureWorks LLC.

### Herstellung des erfindungsgemäßen Carbodiimides CDI (B)

In einem ausgeheizten und mit Stickstoff befüllten 250 ml Vierhalskolben wurden unter Stickstoffstrom 92 g einer Mischung, bestehend aus ca. 20 Gew.% 2,6 Diethyltoluoyldiisocyanat und ca. 80 Gew.% 2,4-Diethyltoluoyldiisocyanat vorgelegt. Nach Zugabe von 50 mg 1-Methyl-phospholenoxid wurde auf 130 °C erwärmt. Im Anschluss wurde bei 130 °C solange carbodiimidisiert bis ein NCO-Gehalt von ca. 12 Gew.% erreicht worden war. Nachdem der Katalysator bei einer Temperatur von ca. 120 °C unter Vakuum vollständig destillativ entfernt wurde, wurde das überschüssige Diisocyanat bei einer Temperatur von ca. 180 °C unter Vakuum abdestilliert. Schließlich wurde die Reaktionsmischung auf ca. 90 - 100°C gekühlt und die endständigen NCO-Gruppen mit PEG 500 MME abreagiert. Das erhaltene Produkt war eine helle, gelbe Flüssigkeit mit einem NCN-Gehalt von ca. 6 Gew.% und einer Polydispersität (Mw/Mn) von ca. 1,5.

### Herstellung des Carbodiimides CDI (C)

In einem ausgeheizten und mit Stickstoff befüllten 250 ml Vierhalskolben wurden unter Stickstoffstrom 150 g einer Mischung, bestehend aus ca. 20 Gew.% 2,6 Diethyltoluoyldiisocyanat und ca. 80 Gew.% 2,4-Diethyltoluoyldiisocyanat vorgelegt. Nach Zugabe von 50 mg 1-Methyl-phospholenoxid wurde auf 180 °C erwärmt. Im Anschluss wurde bei 180 °C solange carbodiimidisiert bis ein NCO-Gehalt von < 1 Gew.% erreicht worden war. Das erhaltene Produkt war eine dunkle, klebrige, erstarrte Schmelze mit einem NCN-Gehalt von ca. 14 Gew.%.

### Herstellung des erfindungsgemäßen Carbodiimides CDI (F) und CDI (G):

In einem ausgeheizten und mit Stickstoff befüllten 250 ml Vierhalskolben wurden unter Stickstoffstrom 92 g einer Mischung, bestehend aus ca. 20 Gew.% 2,6 Diethyltoluoyldiisocyanat und ca. 80 Gew.% 2,4-Diethyltoluoyldiisocyanat vorgelegt. Nach Zugabe von 50 mg 1-Methyl-phospholenoxid wurde auf 130 °C erwärmt. Im Anschluss wurde bei 130 °C solange carbodiimidisiert bis ein NCO-Gehalt von ca. 7 - 9 Gew.% erreicht worden war. Nachdem der Katalysator bei einer Temperatur von ca. 120 °C unter Vakuum vollständig destillativ entfernt wurde, wurde das überschüssige Diisocyanat bei einer Temperatur von ca. 180 °C unter Vakuum abdestilliert. Schließlich wurde die Reaktionsmischung auf ca. 70 - 80 °C gekühlt und die endständigen NCO-Gruppen nach Zugabe von Toluol als Lösemittel mit Ethanol (CDI F) oder Cyclohexanol (CDI G) abreagiert. Nach der destillativen Entfernung des Lösemittels wurden Feststoffe mit einem NCN-Gehalt von ca. 12,5 Gew.% (CDI F) oder 10,2 Gew.% (CDI G) und einer Polydispersität (Mw/Mn) von ca. 1,8 erhalten.

### Thermische Stabilität

Zur Untersuchung der thermischen Stabilität wurden bei 80 °C 1 Gew.% der oben genannten Carbodiimide in Polyesterpolyol mit einer Anfangssäurezahl von ca. 0,9 mg KOH/g eingerührt und 3 h bei dieser Temperatur weiter gerührt. Die Gasphase wurde mittels GC-MS auf Spaltsubstanzen (Isocyanate) untersucht. Die Ergebnisse werden in Tabelle 1 dargestellt:

| **Carbodiimid** | **Nachweis von Spaltsubstanzen (Isocyanate)** |
|---|---|
| CDI (A) (V) | Negativ |
| CDI (B) (erf) | Negativ |

| | |
|---|---|
| V = Vergleichsbeispiel, erf = erfindungsgemäß | |

Aus der Tabelle 1 ist ersichtlich, dass das erfindungsgemäße Carbodiimid CDI (B) eine hervorragende thermische Stabilität aufweist, die mit CDI (A) vergleichbar ist.

### Säurezahlabbau in Polyesterpolyol

Wie bekannt, kann die Wirkung eines Hydrolyseschutzmittels auf Basis sterisch gehinderter Carbodiimide in flüssigen Polyesterpolyolen mittels Säurezahlabbau geprüft werden.

Der Säurezahlabbau in der erfindungsgemäßen Zusammensetzung wurde beim Einsatz des erfindungsgemäßen CDI (B) im Vergleich zu dem aus dem Stand der Technik bekannten CDI (A) einem Polyesterpolyol auf Basis von Adipinsäure (Desmophen® 2001 KS der Bayer MaterialScience AG) geprüft.

Dazu wurden bei 80°C 1 Gew.% der oben genannten Carbodiimide in Polyesterpolyol mit einer anfänglichen Säurezahl von ca. 0,9 mg KOH/g eingerührt und in regelmäßigen Abständen die Säurezahl gemessen.

Die Ergebnisse werden in Tabelle 2 dargestellt:

| **Carbodiimid in Desmophen** ® **2001 KS** | **Säurezahl [mg KOH/g] nach 0 min** | **Säurezahl [mg KOH/g] nach 30 min** | **Säurezahl [mg KOH/g] nach 60 min** | **Säurezahl [mg KOH/g] nach 120 min** | **Säurezahl [mg KOH/g] nach 240 min** | **Säurezahl [mg KOH/g] nach 480 min** |
|---|---|---|---|---|---|---|
| **CDI (B) (erf)** | 0,86 | 0,63 | 0,52 | 0,38 | 0,21 | 0,12 |
| **CDI (A) (V)** | 0,87 | 0,69 | 0,55 | 0,42 | 0,35 | 0,28 |

| | | | | | | |
|---|---|---|---|---|---|---|
| V = Vergleichsbeispiel, erf = erfindungsgemäß | | | | | | |

Die Ergebnisse zeigen, dass die Restsäure des Polyesterpolyols mit dem erfindungsgemäßen Carbodiimid trotz des geringeren NCN-Gehaltes überraschenderweise deutlich schneller abgebaut wird als bei der Zusammensetzung, die das nach dem Stand der Technik bekannt CDI (A) enthält.

### Hydrolyseschutz in Polyethylenterephthalat (PET)

Zur Bewertung der Hydrolyseschutzwirkung in PET wurden je 1,5 Gew.% der untersuchten Carbodiimide mittels eines Labordoppelschneckenextruders ZSK 25 der Firma Werner & Pfleiderer vor der unten beschriebenen Messung in PET eindispergiert. Aus den gewonnenen Granulaten wurden dann die für die Messung der Reißfestigkeit verwendeten F3-Normprüfkörper an einer Spritzgießmaschine des Typs Arburg Allrounder 320 S 150 - 500 hergestellt.

Für den Hydrolysetest wurden diese F3-Normprüfkörper in Wasserdampf bei einer Temperatur von 110°C gelagert und deren Reißfestigkeit in MPa gemessen.

Die Ergebnisse sind in der Tabelle 3 aufgelistet:

| **Reißfestigkeit (MPa)** | **Bsp. 1 (vgl.) (PET)** | **Bsp. 2 (vgl.) (PET, 1 x extrudiert)** | **Bsp. 3 (vgl.) (PET/CDI A)** | **Bsp. 4 (vgl.) (PET/CDI D)** | **Bsp. 5 (erf.) (PET/CDI F)** | **Bsp. 6 (erf.) (PET/CDI G)** |
|---|---|---|---|---|---|---|
| 0 Tage | 84 | 62 | 82 | 88 | 80 | 89 |
| 1 Tag | 73 | 41 | 62 | 76 | 77 | 77 |
| 2 Tage | 72 | 21 | - | - | - | - |
| 3 Tage | 50 | 0 | 2 | 76 | 72 | 71 |
| 4 Tage | 25 | | 0 | 70 | 68 | 68 |
| 5 Tage | 8 | | | 37 | 37 | 46 |

| | | | | | | |
|---|---|---|---|---|---|---|
| vgl. =Vergleichsbeispiel, erf.= erfindungsgemäß | | | | | | |

### Hydrolyseschutz in Polymilchsäure (PLA)

Zur Bewertung der Hydrolyseschutzwirkung in PLA wurden je 1,0 bzw. 1,5 Gew.% der untersuchten Carbodiimide mittels eines Labordoppelschneckenextruders ZSK 25 der Firma Werner & Pfleiderer vor der unten beschriebenen Messung in PLA eindispergiert. Aus den gewonnenen Granulaten wurden dann die für die Messung der Reißfestigkeit verwendeten F3-Normprüfkörper an einer Spritzgießmaschine des Typs Arburg Allrounder 320 S 150 - 500 hergestellt.

Für den Hydrolysetest wurden diese F3-Normprüfkörper in Wasser bei einer Temperatur von 65°C gelagert und deren Reißfestigkeit gemessen.

Die Ergebnisse sind in der Tabelle 4 aufgelistet:

| **Reißfestigkei t (MPA)** | **Bsp. 7 (vgl.) (PLA)** | **Bsp. 8 (vgl.) (PLA, CDI E) 1,0 %/1,5 %** | **Bsp. 9 (vgl.) (PLA, CDI D) 1,0 %/1,5 %** | **Bsp. 10 (erf.) (PET, CDI F) 1,0 %/1,5 %** |
|---|---|---|---|---|
| 0 Tage | 76 | 75/73 | 77/74 | 77/75 |
| 1 Tag | 63 | 67/- | - | - |
| 2 Tage | 47 | 69/- | - | - |
| 3 Tage | 25 | 58/- | 73/73 | 74/75 |
| 4 Tage | 7 | 32/- | 61/71 | - |
| 5 Tage | 0 | - | - | - |
| 7 Tage | | 0/46 | 8/43 | 50/74 |
| 8 Tage | | -/37 | 0/35 | 41/- |
| 9 Tage | | -/19 | -/21 | 23/74 |
| 10 Tage | | -/10 | -/15 | 8/74 |
| 15 Tage | | -/0 | -/0 | 0/63 |
| 18 Tage | | | | -/53 |

| | | | | |
|---|---|---|---|---|
| vgl. =Vergleichsbeispiel, erf.= erfindungsgemäß % = Angaben in Gew.% | | | | |

Die Angaben in der Tabelle 4 entsprechen den Werten zu 1,0 % / 1,5 % des entsprechenden CDI's.

Die Ergebnisse aus den Tabellen 3 und 4 zeigen, dass die erfindungsgemäßen Carbodiimide im Vergleich zum Stand der Technik einen hervorragenden Hydrolyseschutz in PET und PLA zeigen. Darüber hinaus haben diese den Vorteil, dass diese auf einer günstigeren Rohstoffbasis basieren und deutlich weniger aufwändig in der Herstellung sind.

### Versuche zur Pastillierbarkeit und Dosierbarkeit der festen Carbodiimide

Zur Klärung der Konfektionierbarkeit, Handhabung und Dosierbarkeit der verschiedenen festen Carbodiimide wurden diese bezüglich Aussehen, Pastillierbarkeit und Erweichungspunkt verglichen. Die Erweichungspunkte wurden mittels einer Koffler-Bank bestimmt.

Die Ergebnisse sind in der Tabelle 5 aufgelistet:

| Carbodiimid | Aussehen (bei RT) | Pastillierbarkeit | Dosierbarkeit (T bis 40 °C) | Erweichungspunkt (°C) |
|---|---|---|---|---|
| CDI (C), vgl. | harte, klebrige Masse | nicht möglich | nicht möglich | < 20 |
| CDI (D), vgl. | fest, spröde | sehr gut | befriedigend | ca. 50 |
| CDI (E), vgl. | fest, wachsartig | nicht möglich | ausreichend | < 40 |
| CDI (F), erf. | fest, spröde | sehr gut | gut | > 50 |
| CDI (G), erf. | fest, spröde | sehr gut | sehr gut | > 60 |

| | | | | |
|---|---|---|---|---|
| vgl. =Vergleichsbeispiel, erf.= erfindungsgemäß | | | | |

Die Ergebnisse aus der Tabelle 5 zeigen eindeutig, dass die erfindungsgemäßen Carbodiimide auf Basis Diethyltoluoyldiisocyanat endgekappt mit Ethanol oder Cyclohexanol trotz des geringeren Polymerisationsgrades im Vergleich zum polymeren Carbodiimid auf Basis Diethyltoluoyldiisocyanat (nicht endfunktionalisiert) eine hervorragende Pastillierbarkeit und einen hohen Erweichungspunkt aufweisen und damit Vorteile bei der Konfektionierung und Dosierung des Feststoffes bei der Stabilisierung der Ester-basierten Polymere mit sich bringen.

## Patentansprüche

1. Carbodiimide mit endständigen Harnstoff- und/oder Urethangruppen der Formel 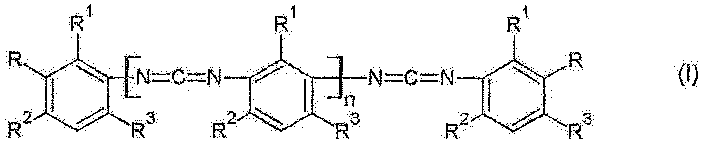
- in der R gleich oder verschieden sein kann und ausgewählt ist aus der Gruppe der -NHCONHR^{I}-, -NHCONR'R"- und -NHCOOR^{III}-Reste, wobei R^{I} und R^{II} gleich oder verschieden sind und einen C₁ - C₂₂ - Alkyl-, C₆ - C₁₂ - Cycloalkyl-, C₆ - C₁₈-Aryl- oder C₆ - C₁₈ -Aralkylrest bedeuten und R^{III} C₁ - C₂₂ - Alkyl, C₆ - C₁₂ -Cycloalkyl, C₆ - C₁₈ -Aryl oder C₆ - C₁₈ -Aralkyl, oder einem ungesättigten Alkylrest mit 2-22, Kohlenstoffatomen, oder einem Alkoxypolyoxyalkylenrest entspricht,
- R¹, R² und R³ jeweils unabhängig für Methyl- oder Ethyl steht, wobei jeder Benzolring nur eine Methyl-Gruppe aufweist und
- n = 0 bis 20 bedeutet.

2. Carbodiimide nach Anspruch 1, **dadurch gekennzeichnet, dass** R ein -NHCOOR^{III}-Rest mit R^{III} ein Alkoxypolyoxyalkylen oder ein ungesättigter Alkylrest mit 18 Kohlenstoffatomen ist, und R¹, R² und R³ jeweils unabhängig für Methyl- oder Ethyl steht, wobei jeder Benzolring nur eine Methyl-Gruppe aufweist und n = 0 bis 20, bevorzugt n = 1 bis 10, besonders bevorzugt n = 1 bis 4, ganz besonders bevorzugt n = 2 bis 3.

3. Carbodiimide nach Anspruch 1, **dadurch gekennzeichnet, dass** R ein -NHCOOR^{III}-Rest, wobei R^{III} ein C₁ - C₂₂ - Alkyl, bevorzugt C₁ - C₆ - Alkyl, besonders bevorzugt Methyl, Ethyl oder i-Propyl, C₆ - C₁₂ -Cycloalkyl, bevorzugt C₆ - Cycloalkyl ist, und R¹, R² und R³ jeweils unabhängig für Methyl- oder Ethyl steht, wobei jeder Benzolring nur eine Methyl-Gruppe aufweist und n = 0 bis 20, bevorzugt n = 1 bis 10, besonders bevorzugt n = 3 bis 8 , bedeutet.

4. Carbodiimide nach einem der Ansprüche 1 bis 2, **dadurch gekennzeichnet, dass** R^{III} ein Polyethylenglykolmonomethylether mit Molmassen von 200 - 600 g/mol, bevorzugt von 350 - 550 g/mol ist.

5. Carbodiimide nach Anspruch 2, **dadurch gekennzeichnet, dass** der NCN-Gehalt im Carbodiimid 2 - 14 Gew.-%, bevorzugt 4 - 10 Gew.-%, besonders bevorzugt 4 - 8 Gew.%, insbesondere bevorzugt 5 - 7 Gew.-% beträgt.

6. Carbodiimide nach Anspruch 3, **dadurch gekennzeichnet, dass** der NCN-Gehalt im Carbodiimid 2 - 14 Gew.-%, bevorzugt 4 - 14 Gew.-%, besonders bevorzugt bei 10-13 Gew.% beträgt.

7. Carbodiimide nach einem der Ansprüche 1 bis 6, **dadurch gekennzeichnet, dass** diese mittlere Molmassen (Mw) von 1000 - 5000 g/mol, bevorzugt 1500 - 4000 g/mol, besonders bevorzugt 2000 - 3000 g/mol aufweisen.

8. Verfahren zur Herstellung der Carbodiimide nach einem der Ansprüche 1 bis 7 **dadurch gekennzeichnet, dass** Diisocyanate der Formel 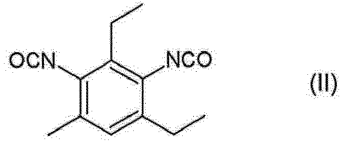 und/oder der Formel 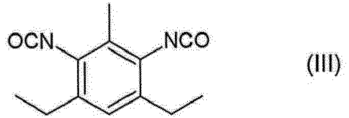 unter Abspaltung von Kohlendioxid bei Temperaturen von 80 °C bis 200 °C in Gegenwart von Katalysatoren und gegebenenfalls Lösungsmittel carbodiimidisiert werden und anschließend die freien NCO-Funktionalitäten mit primären oder sekundären Aminen oder Alkoholen endfunktionalisiert werden.

9. Verfahren zur Herstellung der Carbodiimide nach einem der Ansprüche 1 bis 7 **dadurch gekennzeichnet, dass** zuerst eine partielle, vorzugsweise 50% ige Endfunktionalisierung der freien NCO-Gruppen mit primären oder sekundären Aminen oder Alkoholen und/oder Alkoxypolyoxyalkylenalkoholen in den aromatischen Diisocyanaten der Formel (II) 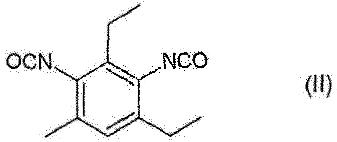 und/oder der Formel (III) 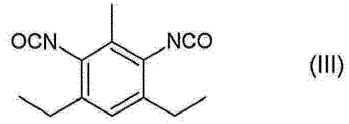 durchgeführt wird und anschließend eine Carbodiimidisierung unter Abspaltung von Kohlendioxid bei Temperaturen von 80 °C bis 200 °C in Gegenwart von Katalysatoren und gegebenenfalls Lösungsmittel erfolgt.

10. Verfahren nach Anspruch 8 **dadurch gekennzeichnet, dass** die Carbodiimidisierung bei Temperaturen von 80 - 200°C, vorzugsweise 120 - 140 °C, vorzugsweise in Gegenwart eines Katalysators durchgeführt wird, und anschließend gegebenenfalls nach Abdestillation des Katalysators bei Temperaturen von 50 - 120 °C und des nicht umgesetzten Diisocyanats, bei Temperaturen von 160 - 180 °C die restlichen NCO-Gruppen des Carbodiimides mit aliphatischen und/oder aromatischen Aminen, Alkoholen und/oder Alkoxypolyoxyalkylenalkoholen, vorzugsweise Polyethylenmonomethylether und/oder Oleyalkohol, bei Temperaturen von 80 - 140 °C gegebenenfalls in Anwesenheit eines PU-Katalysators umgesetzt werden.

11. Verfahren nach Anspruch 8 **dadurch gekennzeichnet, dass** die Carbodiimidisierung bei Temperaturen von 80 - 200°C, vorzugsweise 120 - 140 °C, vorzugsweise in Gegenwart eines Katalysators durchgeführt wird, anschließend zur Unterbrechung der Carbodiimidisierung die Temperatur der Reaktionsmischung auf 50 - 120 °C, bevorzugt 60 - 100 °C, besonders bevorzugt auf 80 - 90 °C reduziert wird und gegebenenfalls nach Zugabe eines Lösemittels, bevorzugt ausgewählt aus der Gruppe der Alkylbenzole, besonders bevorzugt Toluol, die freien endständigen Isocyanatgruppen der Carbodiimide mit aliphatischen und/oder aromatischen Aminen, Alkoholen und/oder Alkoxypolyoxyalkylenalkoholen abreagieren.

12. Verfahren nach einem der Ansprüche 8 bis 9, **dadurch gekennzeichnet, dass** eine Mischung aus Diisocyanaten der Formel (II) und (III) im Verhältnis 70 : 30 bis 9 : 10 carbodiimidisiert wird.

13. Verfahren nach Anspruch 8 zur Herstellung der Carbodiimide nach Anspruch 3, **dadurch gekennzeichnet, dass** die Schmelze nach der Carbodiimidisierung auf Pastillierbändern pastilliert wird.

14. Verfahren nach Anspruch 13, **dadurch gekennzeichnet, dass** bei den Carbodiimiden nach Anspruch 3, R^{III} = Cyclohexyl ist.

15. Zusammensetzung enthaltend
- mindestens ein esterbasiertes Polymer, vorzugsweise ausgewählt aus der Gruppe der Polyesterpolyole, der esterbasierten thermoplastischen Polyurethane, der Polyurethan-Elastomere, der PU-Klebstoffe, der PU-Gießharzen, der Polyethylenterephthalate (PET), der Polybutylenterephthalate (PBT), der Polytrimethylenterephthalate (PTT), Copolyester, der thermoplastischen Polyester-Elastomere (TPE E), der Ethylenvinylacetate (EVA), der Polymilchsäuren (PLA), der Polybutylenadipat-Terephthalate (PBAT), der Polybutylensuccinate (PBS), der PLA-Derivate und/oder der Polyhydroxyalkanoate (PHA)
und
- mindestens ein Carbodiimid nach einem der Ansprüche 1 bis 7.

16. Zusammensetzung nach Anspruch 15 **dadurch gekennzeichnet, dass** die Konzentration des Carbodiimides 0,1 - 5 Gew.%, bevorzugt 0,5 - 3 Gew.%, besonders bevorzugt 1 - 2 Gew.%, beträgt.

17. Verfahren zur Herstellung der Zusammensetzungen nach Anspruch 15 oder 16, **dadurch gekennzeichnet, dass** Carbodiimide nach Anspruch 3 zu den esterbasierten Polymeren ausgewählt aus der Gruppe, umfassend Polyethylenterephthalat (PET), Polybutylenterephthalat (PBT), Polytrimethylenterephthalat (PTT), thermoplastische Polyurethane (TPU), Copolyester, wie das modifizierte Polyester aus Cyclohexandiol und Terephthalsäure (PCTA), thermoplastische Polyester Elastomere (TPE E), Ethylenvinylacetat (EVA), Polymilchsäure (PLA) Polybutylenadipat-Terephthalate (PBAT), Polybutylensuccinate (PBS), PLA-Derivate und/oder Polyhydroxyalkanoate (PHA) mittels Feststoffdosieraggregat zugegeben werden.

18. Verwendung der Carbodiimide nach einem der Ansprüche 1 bis 7 in esterbasierten
Polyolen, in Polyethylenterephthalat (PET), in Polybutylenterephthalat (PBT), in Polytrimethylenterephthalat (PTT), in Copolyestern, in thermoplastischen Polyester-Elastomeren (TPE E), in Ethylenvinylacetat (EVA), in Polymilchsäure (PLA) und/oder in PLA-Derivaten, in Polybutylenadipat-Terephthalaten (PBAT), in Polybutylensuccinaten (PBS), in Polyhydroxyalkanoaten (PHA), in Blends, in Triglyceriden, vorzugsweise Trimethylolpropantrioleat (TMP-Oleat), in Ölformulierungen für die Schmierstoffindustrie, in thermoplastischen Polyurethanen (TPU), in Polyurethan-Elastomeren, in PU-Klebstoffen, in PU-Gießharzen, in PU-Schäumen oder in PU-Beschichtungen für Holz, Leder, Kunstleder und Textilien, als Schutz gegen hydrolytischen Abbau.

19. Verwendung der Carbodiimide nach einem der Ansprüche 1 bis 7 in Polymilchsäure (PLA).

## Claims

1. Carbodiimides having terminal urea and/or urethane groups of the formula 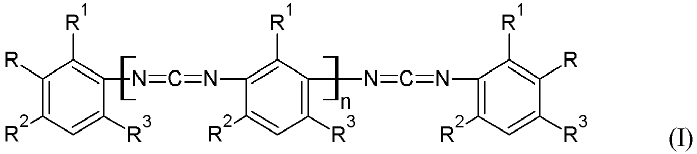
- in which R may be the same or different and is selected from the group of -NHCONHR^{I}, -NHCONR^{I}R^{II} and -NHCOOR^{III} residues, where R^{I} and R^{II} are the same or different and are a C₁ - C₂₂- alkyl, C₆ - C₁₂-cycloalkyl, C₆ - C₁₈-aryl or C₆ - C₁₈-aralkyl residue and R^{III} corresponds to C₁ - C₂₂-alkyl, C₆ - C₁₂-cycloalkyl, C₆ - C₁₈-aryl or C₆ - C₁₈-aralkyl, or an unsaturated alkyl residue having 2-22 carbon atoms, or an alkoxypolyoxyalkylene residue,
- R¹, R² and R³ are each independently methyl or ethyl, wherein each benzene ring has only one methyl group and
- n= 0 to 20.

2. Carbodiimides according to Claim 1, **characterized in that** R is an - NHCOOR^{III} residue where R^{III} is an alkoxypolyoxyalkylene or an unsaturated alkyl residue having 18 carbon atoms, and R¹, R² and R³ are each independently methyl or ethyl, wherein each benzene ring has only one methyl group and n = 0 to 20, preferably n = 1 to 10, particularly preferably n = 1 to 4, especially preferably n = 2 to 3.

3. Carbodiimides according to Claim 1, **characterized in that** R is an - NHCOOR^{III} residue, where R^{III} is a C₁ - C₂₂-alkyl, preferably C₁ - C₆-alkyl, particularly preferably methyl, ethyl or i-propyl, C₆ - C₁₂-cycloalkyl, preferably C₆-cycloalkyl, and R¹, R² and R³ are each independently methyl or ethyl, wherein each benzene ring has only one methyl group and n = 0 to 20, preferably n = 1 to 10, particularly preferably n = 3 to 8.

4. Carbodiimides according to either of Claims 1 and 2, **characterized in that** R^{III} is a polyethylene glycol monomethyl ether with molar masses of 200 - 600 g/mol, preferably 350 - 550 g/mol.

5. Carbodiimides according to Claim 2, **characterized in that** the NCN content in the carbodiimide is 2 - 14% by weight, preferably 4 - 10% by weight, particularly preferably 4 - 8% by weight, more preferably 5 - 7% by weight.

6. Carbodiimides according to Claim 3, **characterized in that** the NCN content in the carbodiimide is 2 - 14% by weight, preferably 4 - 14% by weight, particularly preferably 10 - 13% by weight.

7. Carbodiimides according to any of Claims 1 to 6, **characterized in that** said carbodiimides have average molar masses (Mw) of 1000 - 5000 g/mol, preferably 1500 - 4000 g/mol, particularly preferably 2000 - 3000 g/mol.

8. Method for preparing the carbodiimides according to any of Claims 1 to 7, **characterized in that** diisocyanates of the formula 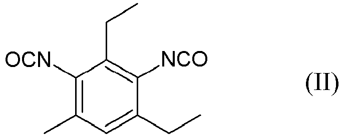 and/or of the formula 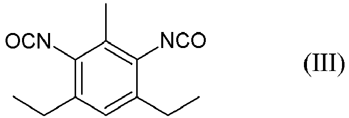 are carbodiimidized with elimination of carbon dioxide at temperatures of 80°C to 200°C in the presence of catalysts and optionally solvent and subsequently the free NCO functionalities are terminally functionalized with primary or secondary amines or alcohols.

9. Method for preparing the carbodiimides according to any of Claims 1 to 7, **characterized in that** initially a partial, preferably <50% terminal functionalization of the free NCO groups in the aromatic diisocyanates of the formula (II) 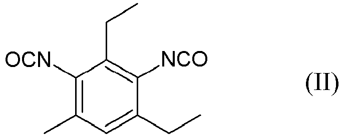 and/or of the formula (III) 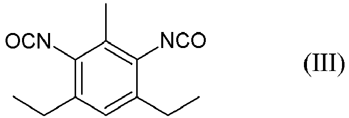 is carried out with primary or secondary amines or alcohols and/or alkoxypolyoxyalkylene alcohols and subsequently a carbodiimidization is carried out with elimination of carbon dioxide at temperatures of 80°C to 200°C in the presence of catalysts and optionally solvent.

10. Method according to Claim 8, **characterized in that** the carbodiimidization is carried out at temperatures of 80 - 200°C, preferably 120 - 140°C, preferably in the presence of a catalyst, and subsequently, optionally after distilling off the catalyst at temperatures of 50 - 120°C and the unreacted diisocyanate at temperatures of 160 - 180°C, the remaining NCO groups of the carbodiimide are reacted with aliphatic and/or aromatic amines, alcohols and/or alkoxypolyoxyalkylene alcohols, preferably polyethylene monomethyl ether and/or oley alcohol, at temperatures of 80 - 140°C, optionally in the presence of a PU catalyst.

11. Method according to Claim 8, **characterized in that** the carbodiimidization is carried out at temperatures of 80 - 200°C, preferably 120 - 140°C, preferably in the presence of a catalyst, subsequently the temperature of the reaction mixture is reduced to 50 - 120°C, preferably 60 - 100°C, particularly preferably to 80 - 90°C to interrupt the carbodiimidization and, optionally after addition of a solvent, preferably selected from the group of alkylbenzenes, particularly preferably toluene, the free terminal isocyanate groups of the carbodiimides are reacted with aliphatic and/or aromatic amines, alcohols and/or alkoxypolyoxyalkylene alcohols.

12. Method according to either of Claims 8 and 9, **characterized in that** a mixture of diisocyanates of the formula (II) and (III) is carbodiimidized in a ratio of 70 : 30 to 90 : 10.

13. Method according to Claim 8 for preparing the carbodiimides according to Claim 3, **characterized in that**, after the carbodiimidization, the melt is pelletized on pelletizing belts.

14. Method according to Claim 13, **characterized in that** in the carbodiimides according to Claim 3, R^{III} = cyclohexyl.

15. Composition comprising
- at least one ester-based polymer, preferably selected from the group of polyester polyols, ester-based thermoplastic polyurethanes, polyurethane elastomers, PU adhesives, PU casting resins, polyethylene terephthalates (PET), polybutylene terephthalates (PBT), polytrimethylene terephthalates (PTT), copolyesters, thermoplastic polyester elastomers (TPE E), ethylene vinyl acetates (EVA), polylactic acids (PLA), polybutylene adipate terephthalates (PBAT), polybutylene succinates (PBS), PLA derivatives and/or polyhydroxyalkanoates (PHA)
and
- at least one carbodiimide according to any of Claims 1 to 7.

16. Composition according to Claim 15, **characterized in that** the concentration of the carbodiimide is 0.1 - 5% by weight, preferably 0.5 - 3% by weight, particularly preferably 1 - 2% by weight.

17. Method for preparing the compositions according to Claim 15 or 16, **characterized in that** carbodiimides according to Claim 3 are added by means of a solids metering unit to the ester-based polymers selected from the group comprising polyethylene terephthalate (PET), polybutylene terephthalate (PBT), polytrimethylene terephthalate (PTT), thermoplastic polyurethanes (TPU), copolyesters such as the modified polyester of cyclohexanediol and terephthalic acid (PCTA), thermoplastic polyester elastomers (TPE E), ethylene vinyl acetate (EVA), polylactic acid (PLA), polybutylene adipate terephthalate (PBAT), polybutylene succinates (PBS), PLA derivatives and/or polyhydroxyalkanoates (PHA) .

18. Use of the carbodiimides according to any of Claims 1 to 7 as protection against hydrolytic degradation in ester-based polyols, in polyethylene terephthalate (PET), in polybutylene terephthalate (PBT), in polytrimethylene terephthalate (PTT), in copolyesters, in thermoplastic polyester elastomers (TPE E), in ethylene vinyl acetate (EVA), in polylactic acid (PLA) and/or in PLA derivatives, in polybutylene adipate terephthalates (PBAT), in polybutylene succinates (PBS), in polyhydroxyalkanoates (PHA), in blends, in triglycerides, preferably trimethylolpropane trioleate (TMP oleate), in oil formulations for the lubricants industry, in thermoplastic polyurethanes (TPU), in polyurethane elastomers, in PU adhesives, in PU casting resins, in PU foams or in PU coatings for wood, leather, artificial leather and textiles.

19. Use of the carbodiimides according to any of Claims 1 to 7 in polylactic acid (PLA).

## Revendications

1. Carbodiimides contenant des groupes urée et/ou uréthane terminaux de formule 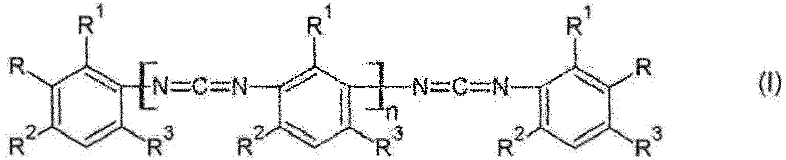
- dans laquelle les R peuvent être identiques ou différents, et sont choisis dans le groupe constitué par les radicaux -NHCONHR^{I}-, -NHCONR^{I}R^{II}- et -NHCOOR^{III}-, R^{I} et R^{II} étant identiques ou différents et signifiant un radical alkyle en C₁-C₂₂, cycloalkyle en C₆-C₁₂, aryle en C₆-C₁₈ ou aralkyle en C₆-C₁₈, et R^{III} correspondant à un radical alkyle en C₁-C₂₂, cycloalkyle en C₆-C₁₂, aryle en C₆-C₁₈ ou aralkyle en C₆-C₁₈ ou à un radical alkyle insaturé de 2 à 22 atomes de carbone ou à un radical alcoxypolyoxyalkylène,
- R¹, R² et R³ représentent chacun indépendamment méthyle ou éthyle, chaque cycle benzène ne comprenant qu'un groupe méthyle, et
- n = 0 à 20.

2. Carbodiimides selon la revendication 1, **caractérisés en ce que** R est un radical -NHCOOR^{III}- avec R^{III} un radical alcoxypolyoxyalkylène ou alkyle insaturé de 18 atomes de carbone, et R¹, R² et R³ représentent chacun indépendamment méthyle ou éthyle, chaque cycle benzène ne comprenant qu'un groupe méthyle, et n = 0 à 20, de préférence n = 1 à 10, de manière particulièrement préférée n = 1 à 4, de manière tout particulièrement préférée n = 2 à 3.

3. Carbodiimides selon la revendication 1, **caractérisés en ce que** R est un radical -NHCOOR^{III}- avec R^{III} un radical alkyle en C₁-C₂₂, de préférence alkyle en C₁-C₆, de manière particulièrement préférée méthyle, éthyle ou i-propyle, cycloalkyle en C₆-C₁₂, de préférence cycloalkyle en C₆, et R¹, R² et R³ représentent chacun indépendamment méthyle ou éthyle, chaque cycle benzène ne comprenant qu'un groupe méthyle, et n = 0 à 20, de préférence n = 1 à 10, de manière particulièrement préférée n = 3 à 8.

4. Carbodiimides selon l'une quelconque des revendications 1 à 2, **caractérisés en ce que** R^{III} est un éther monométhylique de polyéthylène glycol ayant des masses molaires de 200 à 600 g/mol, de préférence de 350 à 550 g/mol.

5. Carbodiimides selon la revendication 2, **caractérisés en ce que** la teneur en NCN dans le carbodiimide est de 2 à 14 % en poids, de préférence de 4 à 10 % en poids, de manière particulièrement préférée de 4 à 8 % en poids, de manière notamment préférée de 5 à 7 % en poids.

6. Carbodiimides selon la revendication 3, **caractérisés en ce que** la teneur en NCN dans le carbodiimide est de 2 à 14 % en poids, de préférence de 4 à 14 % en poids, de manière particulièrement préférée de 10 à 13 % en poids.

7. Carbodiimides selon l'une quelconque des revendications 1 à 6, **caractérisés en ce que** ceux-ci présentent des masses molaires moyennes (Mw) de 1 000 à 5 000 g/mol, de préférence de 1 500 à 4 000 g/mol, de manière particulièrement préférée de 2 000 à 3 000 g/mol.

8. Procédé de fabrication des carbodiimides selon l'une quelconque des revendications 1 à 7, **caractérisé en ce que** des diisocyanates de formule 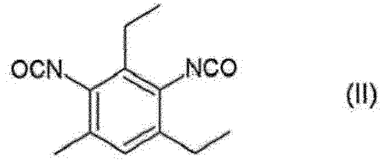 et/ou de formule 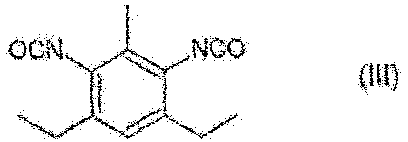 sont carbodiimidés avec clivage de dioxyde de carbone à des températures de 80 °C à 200 °C en présence de catalyseurs et éventuellement de solvants, puis les fonctionnalités NCO libres sont soumises à une fonctionnalisation terminale avec des amines ou des alcools primaires ou secondaires.

9. Procédé de fabrication des carbodiimide selon l'une quelconque des revendications 1 à 7, **caractérisé en ce qu'**une fonctionnalisation terminale partielle, de préférence < 50 %, des groupes NCO libres est tout d'abord réalisée avec des amines ou des alcools primaires ou secondaires et/ou des alcoxypolyoxyakylène-alcools dans les diisocyanates aromatiques de formule (II) 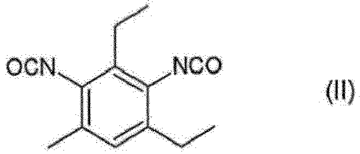 et/ou de formule (III) 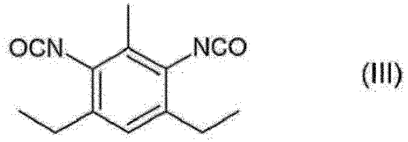 puis une carbodiimidation a lieu avec clivage de dioxyde de carbone à des températures de 80 °C à 200 °C en présence de catalyseurs et éventuellement de solvants.

10. Procédé selon la revendication 8, **caractérisé en ce que** la carbodiimidation est réalisée à des températures de 80 à 200 °C, de préférence de 120 à 140 °C, de préférence en présence d'un catalyseur, puis, éventuellement après l'élimination par distillation du catalyseur à des températures de 50 à 120 °C et du diisocyanate non réagi, à des températures de 160 à 180 °C, les groupes NCO restants du carbodiimide sont mis en réaction avec des amines aliphatiques et/ou aromatiques, des alcools et/ou des alcoxypolyoxyalkylène-alcools, de préférence l'éther monométhylique de polyéthylène et/ou l'alcool oléylique, à des températures de 80 à 140 °C, éventuellement en présence d'un catalyseur de PU.

11. Procédé selon la revendication 8, **caractérisé en ce que** la carbodiimidation est réalisée à des températures de 80 à 200 °C, de préférence de 120 à 140 °C, de préférence en présence d'un catalyseur, puis, pour l'interruption de la carbodiimidation, la température du mélange réactionnel est réduite à une température de 50 à 120 °C, de préférence de 60 à 100 °C, de manière particulièrement préférée de 80 à 90 °C, et éventuellement après l'ajout d'un solvant, de préférence choisi dans le groupe des alkylbenzènes, de manière particulièrement préférée le toluène, les groupes isocyanate terminaux libres des carbodiimides sont mis en réaction avec des amines aliphatiques et/ou aromatiques, des alcools et/ou des alcoxypolyoxyalkylène-alcools.

12. Procédé selon l'une quelconque des revendications 8 à 9, **caractérisé en ce qu'**un mélange de diisocyanates de formule (II) et (III) est carbodiimidé en un rapport de 70:30 à 90:10.

13. Procédé selon la revendication 8 pour la fabrication des carbodiimides selon la revendication 3, **caractérisé en ce que** la masse fondue est pastillée après la carbodiimidation sur des bandes de pastillage.

14. Procédé selon la revendication 13, **caractérisé en ce que**, dans les carbodiimides selon la revendication 3, R^{III} = cyclohexyle.

15. Composition contenant:
- au moins un polymère à base d'ester, de préférence choisi dans le groupe constitué par les polyester-polyols, les polyuréthanes thermoplastiques à base d'ester, les élastomères de polyuréthane, les adhésifs de PU, les résines à couler de PU, les polyéthylène téréphtalates (PET), les polybutylène téréphtalates (PBT), les polytriméthylène téréphtalates (PTT), les copolyesters, les polyesters élastomères thermoplastiques (TPE-E), l'éthylène-acétate de vinyle (EVA), les acides polylactiques (PLA), les polyadipate-téréphtalates de butylène (PBAT), les polysuccinates de butylène (PBS), les dérivés de PLA et/ou les polyhydroxyalcanoates (PHA),
et
- au moins un carbodiimide selon l'une quelconque des revendications 1 à 7.

16. Composition selon la revendication 15, **caractérisée en ce que** la concentration du carbodiimide est de 0,1 à 5 % en poids, de préférence de 0,5 à 3 % en poids, de manière particulièrement préférée de 1 à 2 % en poids.

17. Procédé de fabrication des compositions selon la revendication 15 ou 16, **caractérisé en ce que** des carbodiimides selon la revendication 3 sont ajoutés aux polymères à base d'ester choisis dans le groupe comprenant le polyéthylène téréphtalate (PET), le polybutylène téréphtalate (PBT), le polytriméthylène téréphtalate (PTT), les polyuréthanes thermoplastiques (TPU), les copolyesters, tels que le polyester modifié de cyclohexanediol et d'acide téréphtalique (PCTA), les polyesters élastomères thermoplastiques (TPE-E), l'éthylène-acétate de vinyle (EVA), l'acide polylactique (PLA), le polyadipate-téréphtalate de butylène (PBAT), le polysuccinate de butylène (PBS), les dérivés de PLA et/ou les polyhydroxyalcanoates (PHA), au moyen d'un appareil d'ajout de solides.

18. Utilisation des carbodiimides selon l'une quelconque des revendications 1 à 7 dans des polyols à base d'ester, dans le polyéthylène téréphtalate (PET), dans le polybutylène téréphtalate (PBT), dans le polytriméthylène téréphtalate (PTT), dans des copolyesters, dans des polyesters élastomères thermoplastiques (TPE-E), dans l'éthylène-acétate de vinyle (EVA), dans l'acide polylactique (PLA) et/ou dans des dérivés de PLA, dans des polyadipate-téréphtalates de butylène (PBAT), dans des polysuccinates de butylène (PBS),dans des polyhydroxyalcanoates (PHA), dans des mélanges, dans des triglycérides, de préférence le trioléate de triméthylolpropane (oléate de TMP), dans des formulations huileuses pour l'industrie des lubrifiants, dans des polyuréthanes thermoplastiques (TPU), dans des élastomères de polyuréthane, dans des adhésifs de PU, dans des résines à couler de PU, dans des mousses de PU ou dans des revêtements de PU pour le bois, le cuir, le cuir artificiel et les textiles, en tant que protection contre la décomposition hydrolytique.

19. Utilisation des carbodiimides selon l'une quelconque des revendications 1 à 7 dans l'acide polylactique (PLA).
